# EUROPEAN PATENT APPLICATION

(11) **EP 1 418 164 A1**
(43) Date of publication of application: **12.05.2004**
(21) Application number: 02292786.7
(22) Date of filing: 07.11.2002
(51) Int. Cl.: C07C 25/28, C07C 39/21, C07C 39/373, C07C 43/215, C07C 43/225, C07C 43/23, A61P 17/00, A61P 19/10, A61P 31/12, A61P 31/18, A61K 31/035, A61K 31/05, A61K 31/055, A23L 1/03, A24D 3/14

(54) **New stilbene derivatives and their use as aryl hydrocarbon receptor ligand antagonists**

(71) Applicant: INSTITUT NATIONAL DE LA SANTE ET DE LA RECHERCHE MEDICALE (INSERM), 75654 Paris Cédex 13 (FR)
(72) Inventor: Savouret, Jean-Francois, 94240 L'HAY LES ROSES (FR); Poirot, Marc, 31240 L'UNION (FR); De Medina, Philippe, 31130 BALMA (FR); Casper, Robert F., Toronto, Ontario, M5S 2X9 (CA)
(74) Representative: Peaucelle, Chantal

(57) **Abstract**

The invention relate to stilbene derivatives having formula I 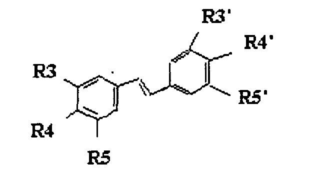 wherein
R3, R4 and R5 and R3', R4' and R5' are identical or different and represent H, OH, O-alkoxy or hal, said alkoxy group being a C1-C6 alkoxy and "hal" being F, Cl or CF₃, with the proviso that one of R4', R3 and R5 or R4, R3' and R5' does not represent OH, OCH₃ or OCH₂ CH₃ when the two other subtituents are both OH, OCH₃, OCH₂CH₃, respectively,
and the symmetrical derivatives.

Use of said stilbene derivatives particularly as pharmaceutical compositions and food additives.

## Description

The invention relates to new stilbene derivatives having, particularly, specific antagonistic ligand properties with respect to the Aryl Hydrocarbon Receptor (AhR) and their use for the prevention and treatment of poisoning and pathologies caused by toxic aryl hydrocarbons and other ligands of the AhR.

Halogenated (HAH) and polycyclic (PAH) aryl hydrocarbons, polyaromatic hydrocarbons, polychlorinated biphenyls (PCB) and other industrial chemicals that bind to the aryl hydrocarbon receptor (AhR) are environmental contaminants which will be collectively referred to hereinafter by the term AhR ligands.

AhR ligands have attracted much attention and concern recently because of their resistance to degradation, resulting in a long biologic half-life (> 10 years) in soil (see ref. 1) and between 4 and 12 years in human blood and fat (2).

Every person on earth is continually exposed to AhR ligands which are present in cigarette smoke, in exhaust fumes from both gasoline and diesel engines, in furnace gases, in cooked meat and fish, in dairy products, and even in mother's milk.

There is sufficient evidence to link exposure to AhR ligands to the development of numerous pathologies such as atherosclerosis, cancer, immunosuppression, skin disorders, reproductive failure, and diminished resistance to viral infection.

Attempts to reduce exposure to said environmental toxins have not been successful, nor are they likely to be until internal combustion engines, the use of fossil fuels, and cigarette smoking are eliminated.

It is, therefore, imperative to develop methods to antagonize the adverse effects of toxic AhR ligands. In WO 99/56737 in the name of INSERM, co-inventors of the present patent application disclosed the potent anti-dioxin effects of resveratrol and analogs and gave a valid response to this challenge.

The inventors have elaborated new stilbene derivatives and have surprisingly found more active and more selective AhR antagonists than resveratrol.

Advantageously contrary to resveratrol, such derivatives are devoid of affinity for the estrogen receptor (ER).

Accordingly, the primary object of the invention is to provide new stilbene derivatives with antagonist properties with respect to AhR ligands.

Another object of the invention is to provide drugs for preventing or treating pathologies and poisoning induced by exposure to AhR ligands.

The stilbene derivatives of the invention have formula I : 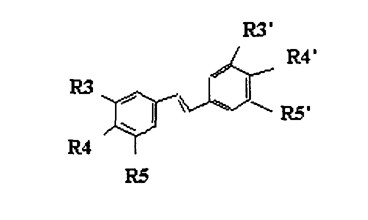 wherein
R3, R4 and R5 and R3', R4' and R5' are identical or different and represent H, OH, O-alkoxy or hal, said alkoxy group being a C1-C6 alkoxy and "hal" being F, Cl or CF₃, with the proviso that one of R4', R3 and R5 or R4, R3' and R5' does not represent OH, OCH₃, or OCH₂ CH₃ when the two other substituents are OH, OCH₃, or OCH₂ CH₃, respectively,
and the symmetrical derivatives.

As shown by the results given in the Examples, said derivatives have a high affinity for the AhR, but without detectable affinity for the ER.

Said derivatives are in racemic form or in the form of geometric cis or trans isomers.

Preferred stilbene derivatives are trans isomers.

In a preferred group, R3 and R5 are hal.

In advantageous derivatives of said group R3' or R4' is hal, alkoxy or hydroxy, and R5'is H.

Preferably R3 and R5 are Cl and in particularly preferred derivatives R3' is H and R4' or R5' is methoxy or Cl.

The invention particularly relates to (E)-1-(4'-trifluoromethylphenyl)-2-(3,5-ditrifluoromethylphenyl)-ethene, (E)-1-(4'-methoxyphenyl)-2-(3,5-dichlorophenyl)-ethene, and (E)-1-(4'-chlorophenyl)-2-(3,5-dichlorophenyl)-ethene which bound to AhR with respective relative binding affinity (RBA) of 52.1, 112.0 and 130.0 without detectable affinity for ER.

The invention also relates to the symmetrical compounds, the positions 4'-3,5 and 4-3',5' with respect to said substituents being identical. The invention particularly relates to the derivatives wherein R3',R5' and R4 represent hal or R3' and R5' are hal and R4 is OH or alkoxy.

Advantageously, said compounds are not toxic on cell culture up to 10µM.

They are particularly useful to investigate further the role of AhR both at the physiological level and in pathologies in which this receptor is involved through activation by xenobiotics.

Said derivatives are useful as antagonists to AhR ligands for binding to the AhR receptor.

Their absence of toxicity make them of interest as active principle in therapeutic and nutritional fields, and in general for the prevention or treatment of disorders due to the toxic effects resulting from exposure to AhR ligands.

The pharmaceutical compositions of the invention comprise an effective amount of at least one stilbene derivative as above defined with pharmaceutically acceptable carrier.

Said pharmaceutical compositions are advantageously in a form for administration by the oral, nasal, parenteral or topical route.

The drugs thus developped are presented in forms appropriate for administration by the oral route, such as drops, gel capsules, syrup or alcohol syrup, by the nasal route, such as spray or drops, by the parenteral route, in the form of a solution in an appropriate solvent, or again by the topical route, such as cream, ointment, shampoo or lotion. The pharmaceutically acceptable vehicles or excipients used are based on oils authorized in the pharmacopoeia, or on an alcohol such as ethanol or propylene-glycol (from 10% to 100%) or on DMSO. In general, they are non-aqueous solvents. Use can also be made of a lipid preparation such as Intralipid^{R}.

It will be noted that said antagonistic stilbenes are soluble in the excipients and/or vehicles used for the preparation of drugs.

The dosage in the different forms and for daily administration will be established, in the standard way, depending on the type of effects to be treated.By way of example, the said drugs based on the said antagonists will be administered in an amount of from 0.1 mg to 500 mg/day, especially from 20 mg to 200 mg/day, and in particular from 10 to 100 mg/day.

Higher doses of stilbene derivatives, up to 5 g per day, can be administered as an antidote for acute intoxication by AhR ligands.

The drugs developed on the basis of these antagonists can be used in various pathologies involving AhR ligands, such as atherogenesis, immunosuppression, cancer, viral infections such as AIDS, allergies and dermatological diseases associated with dioxin and the AhR ligands and with osteoporosis.

The inhibitory effects of the said stilbenes towards the AhR ligands are also advantageously exploited in dietetics.

The invention also concerns the use of the above-defined antagonists as food additives.

Thus it concerns foods characterized in that they contain at least one antagonist such as defined above in a quantity allowing it to exert an inhibitory effect towards AhR ligands, so as to prevent their harmful effects. These foods are intended for adults or for children and infants.

These additives are incorporated in the food, for example in powdered milk, liquid or solid preparations (cereals) or canned foods.

They can also be dissolved in oil for administration as drops or as a food additive.

They can be incorporated during the production of the foods or when they are packaged, or by using preparations with the antagonists already formulated, making it possible to effect the desired supplementation.

The dose used will be reduced compared to that adopted for an adult, taking account of the weight of the child and its food, so as to obtain an effective plasma concentration (of the order of micromolar).

The antagonists of the invention are likewise of great value for combating the effects of smoking, which corresponds to chronic exposure to AhR ligands.

The advantageous properties of the antagonists of the invention are exploited by using them to impregnate cigarette filters, so as to deliver a dose of stilbene derivatives proportional to the concentration of the poisons absorbed with the smoke, and leading to a blood concentration making it possible to inhibit toxic effects of AhR ligands.

Other characteristics and advantages of the invention are given in the examples that follow, which, by way of illustration.

### Experimental Section

**Chemicals :** 2,3,7,8-tetrachloro (1,6-3H) dibenzo-p-dioxin, 28 Ci/mmole was purchased from Terrachem (Lenexa, KS). Dioxin stock solutions were initially dissolved in dimethylsulfoxide and handled under a fume hood. TCDD stock was subsequently diluted in ethanol for use in experiments described below. Steroids were purchased from Steraloids (Wilton, NH). All other chemicals were purchased from Sigma Chemicals.

### Chemistry

1H NMR were recorded at 200 MHz on a Brucker AC200 spectrometer. Chemical shifts are given in parts per million and tetramethylsilane was used as the internal standard for spectra obtained in acetone-d₆. All J values are given in Hz. Mass spectra were recorded on a variant MAT 311 A mass spectrometer. Elementar analysis was carried out by the microanalytical service laboratory of the Ecole Supérieure de Chimie of the Université Paul Sabatier, France and all values were within ±0.4% of the calculated composition. Reagents and solvents were used as obtained from commercial suppliers without further purification. Reaction progress was determined by TLC analysis on silica gel coated aluminium plates. Visualization with UV light (254 nM). All stilbene were purified by RP HPLC and detected by UV light (254 nM). Melting points were uncorrected and measured on a Kofler apparatus. Phosphonium chloride **2a-g** were prepared by refluxing a stirred mixture of triphenylphosphine and the corresponding benzyl chloride in toluene. Yields were better than 50% for all experiments.

### General procedure for the preparation of stilbenes (Z) and stilbenes (E)

Phosphonium salt (1mmol) was dissolved in dichloromethane(1ml). Aryl aldehyde (1mmol), 18 crown 6 (0,1 mmol) and potassium hydroxyde (3mmol) was added to the solution. Reaction mixture was stirred at room temperature and periodically monitored by TLC (toluene or hexane) until complete consumption of the aldehyde. The mixture was dilute with dichloromethane and filtered. The organic layer was washed with, dried over MgSO₄ and evaporated under vacuum. Purification by RP HPLC (Ultrasep ES 100 RP 18, 250 x 8 mm, 6.0 µm; methanol :water, 80:20 or 85:15 ; flow rate 1ml/min) afford pure product.

### (E)-1-(4-methoxyphenyl)-2-(3,5-dimethoxyphenyl)-ethene (4a)

Mp : 55-57°C ; Rf (toluene) : 0,4 ; ¹H NMR (acetone-*d*₆) 3.808 (s, 6H), 3.813 (s, 3H), 6.387 (t, J=2.25Hz, 1H), 6.743 (d, J=2.5Hz, 2H ), 6.94 (m, 2H), 7.05 (d, J=16.5Hz, 1H), 7.17 (d, J=16.5Hz, 1H), 7.53 (m, 2H); ¹³C NMR (acetone-*d*₆): 55.575, 100.245, 105.056, 114.957, 127.285, 128.661, 129.463, 130.876, 140.746, 160,489, 162.081; MS (DCI/NH3) : *m*/*z* 271 (M+1); UV : λ₁= 307 nm ε= 24 600 M⁻¹.cm⁻¹, λ₂= 317 nm ε= 23 800 M⁻¹.cm⁻¹

### (E)-1-(4-chlorophenyl)-2-(3,5-dichlorophenyl)-ethene (4b)

Mp: 94-96°C ;Rf (hexane): 0.56; ¹H NMR (acetone-*d*₆): 7.257 (d, 1H, J= 16.44Hz, 1H ), 7.369 (t, J= 1.9 Hz, 1H), 7.4-7.49 (m, 2H), 7.448 (d, 1H, J= 16.32Hz, 1H), 7.62 (d, J= 1.9 Hz, 2H), 7.625-7.68 (m, 2H);¹³C NMR (acetone-*d*₆): 125.84, 127.361, 127.77, 129.292, 129.727, 135.872; MS (DCI/CH₄): *m*/*z*: 247 (MH⁺-HCl;47), 283 (MH⁺;100), 311 (M....C₂H₅⁺;35), 323(M....C₃H₅⁺;2.2); UV : λ₁= 301 nm ε= 20 500 M⁻¹.cm⁻¹,λ₂= 313 nm ε = 20 500 M⁻¹.cm⁻¹.

### (E)-1-(4-methoxyphenyl)-2-(3,5-difluorophenyl)-ethene (4c)

Mp: 108-110°C; Rf ( toluene 1 / hexane 1) : 0.58; ¹H NMR (acetone-*d*₆): 3.826 (s, 3H ), 6.85 (tt, 1H, J₁=10.1 Hz, J₂=2.3 Hz, 1H), 6.96 (m, 2H), 7.57 (m, 2H), 7.09 (d, J = 16.4 Hz, 1H), 7.34 (d, J = 16.4 Hz, 1H), 7.21 (dt, J₁=7.14 Hz, , J₂= 1.6 Hz, 2H); ¹³C NMR (acetone-*d*₆): 55,619, 102.607, 109.56, 115.052, 124.834, 129.115, 130.119, 132.122, 142.894, 161.011, 164.23; MS (DCI/CH₄): *m*/*z*=227 (MH⁺-HF;15.8), 246 (M⁺;100), 275 (M....C₂H₅⁺; 13.5);UV : λ₁= 308 nm ε= 46 320 M⁻¹.cm⁻¹,λ₂= 320 nm ε= 49 800 M⁻¹.cm⁻¹.

### (E)-1-(4-fluorophenyl)-2-(3,5-difluorophenyl)-ethene (4d)

Mp : 47 °C ; Rf (hexane) :0.34; ¹H NMR (acetone-*d*₆): 6.91 (tt, J₁= 9.5 Hz, J₂=2.2Hz, 1H), 6.743(d, 2H, J= 2.5Hz, 2H ),7.17-7.28 (m, 5H), 7.41 (d, J=16.42Hz, 1H), 7.68 (m, 2H);¹³C NMR (acetone-*d*₆): 103.1, 109.9, 116.445, 127.123, 129.63,131.23, 142.364, 163.56, 164.2 MS (DCI/CH₄): *m*/*z*=215 (MH⁺-HF; 38), 234 (M⁺;100), 263 (M....C₂H₅⁺; 23.47), 275 (M....C₃H₅⁺; 1.04); UV : λ₁= 295 nm ε= 23 900 M⁻¹.cm⁻¹, λ₂= 307 nm ε= 22 100 M⁻¹.cm⁻¹

### (E)-1-(4-trifluoromethylphenyl)-2-(3,5-ditrifluoromethylphenyl)-ethene (4e)

Mp :99-100°C ;Rf(hexane) : 0.39 ; ¹H NMR (acetone-*d*₆): 7.65 (d, J=16.6 Hz, 1H ), 7.77 (d, J=16.6 Hz, 1H ), 7.76 (m, 2H), 7.91 (m, 2H), 7.96 (s,1H), 8 31 (s,2H) ; ¹³C NMR (acetone d₆) 121.870, 126.541, 127.857, 128.371, 129.136, 132.051; MS ( DCI/CH4): *m*/*z* = 365(MH⁺-HF ;100), 385 (MH⁺ ; 23.58), 413 (M....C₂H₅⁺; 12.5), 425 (M....C₃H₅⁺; 1.95); UV : λ₁= 297 nm ε= 28 200 M⁻¹.cm⁻¹, λ₂= 307 nm ε= 25 800 M⁻¹.cm⁻¹

### (E)-1-(4-fluorophenyl)-2-(3,5-dimethoxyphenyl)-ethene (4f)

Mp: 46-48°C; Rf ( toluene): 0.53; ¹H NMR (acetone-*d*₆): 3.81 (s, 6H), 6.42 ( t, J=2.25 Hz, 1H), 6.77 (d, J=2.26 Hz, 2H), 7.08-7.182 (m, 3H), 7.269 (d, J= 16.4Hz, 1H ), 7.635 (m, 2H); ¹³C NMR: 55.601, 100.666, 105.347, 116.28, 128.591, 129.172, 129.522; MS (DCI/NH3): *m*/*z* = 259 (MH⁺ ; 100) ;UV : λ₁= 297 nm ε= 22 900 M⁻¹.cm⁻¹ ,λ₂= 308 nm ε= 21 200 M⁻¹.cm⁻¹

### (E)-1-(4-ethoxyphenyl)-2-(3,5-dimethoxyphenyl)-ethene (4g)

Mp: 54-55°C; Rf (toluene): 0.45; ¹H NMR (acetone-*d*₆): 1.366 ( t, J= 6.955 Hz, 3H), 3.808(s, 6H), 4.06 (q, J= 6.98 Hz, 2H), 6.385 (t, J=2.25 Hz, 1H), 6.741 (d, J=2.23 Hz, 2H), 6.44(d, J= 16.1 Hz, 1H ), 6.55 ( d, J= 16.1 Hz, 1H ), 6.918(m, 2H), 7.513(m, 2H); ¹³C NMR (acetone-*d*₆): 15.094, 55,564, 64.012, 100.196, 105.022 , 115.441, 127.157, 128.661, 129.492, 130.725, 140.757, 159.812, 162.07; MS (DCI/NH3): *m*/*z* 285 (M+1, 100); UV : λ₁= 307 nm ε= 36 900 M⁻¹.cm⁻¹, λ₂= 320 nm ε= 36 800 M⁻¹.cm⁻¹

### (E)-1-(4-butoxyphenyl)-2-(3,5-dimethoxyphenyl)-ethene (4h)

Mp: 54-56°C; Rf (toluene): 0.51; ¹H NMR (acetone-*d*₆): 0.9666 (t, J= 7.28Hz, 3H), 1.49 (m,2H) 1.755 (m,2H), 3.808 (s,6H), 4.007 (t, J= 6.35Hz ,2H), 6.3866 (t, J=2.24 Hz, 1H), 6.742 (d, J=2.25 Hz, 2H), 6.927 (m, 2H), 7.512 (m, 2H) 7.016 (d, J= 16.36 Hz, 1H ), 7.2 (d, J= 16.36 Hz, 1H ); ¹³C NMR (acetone-*d*₆): 14.109, 19.888, 32.076, 55,566, 68.256, 100.206, 105.035, 115.485, 127.148, 128.653, 129.505, 130.712, 140.76, 159.995, 162.075 ; MS (DCI/NH3): *m*/*z* 313 (M+1, 100); UV : λ₁= 307 nm ε= 40 100 M⁻¹.cm⁻¹ ,λ₂= 320 nm ε= 40 000 M⁻¹.cm⁻¹.

### (E)-1-(4-trifluoromethylphenyl)-2-(3,5-dichlorophenyl)-ethene (4i)

Mp: 98-100°C; Rf (hexane): 0.43; ¹H NMR (acetone-*d*₆): 7.395 (d, J= 16.54 Hz, 1H ), 7.561 (d, J= 16.54 Hz, 1H ), 7.4107 (t, J=1.87 Hz,1H), 7.674 (d, J=1.84 Hz, 2H), 7.74 (m, 2H), 7.86 (m, 2H); ¹³C NMR (acetone-*d*₆): 126.116, 126.569, 128.223, 129.352, 131.098, 135.93 MS (DCI/CH4): *m*/*z* 281 (MH⁺-HCl ;11.8), 297 (MH⁺-HF ;100), 317 (MH⁺ ;79.7), 345 (M....C₂H₅⁺; 24.8); UV : λ₁= 297 nm ε= 41 000 M⁻¹.cm⁻¹ ,λ₂= 309 nm ε= 41 100 M⁻¹.cm⁻¹ ,λ₃= 324 nm ε= 25 000 M⁻¹.cm⁻¹

### (E)-1-(4-methoxyphenyl)-2-(3,5-dichlorophenyl)-ethene (4j)

Mp: 62-64°C; Rf ( toluene/hexane): 0.7; ¹H NMR (acetone-*d*₆): 3.828 (s, 3H), 6.96 (m, 2H), 7.07 ); ¹³C NMR (acetone-*d*₆): 55.621, 115.045, 124.118, 125.382, 127.011, 129.161, 132.419; MS (DCI/NH3): *m*/*z* 278 (MH⁺ ;100), 296 (MH⁺....NH3; 15.4); UV : λ₁= 309 nm ε= 44 400 M⁻¹.cm⁻¹ ,λ₂= 325 nm ε= 51 600 M⁻¹.cm⁻¹

### (Z)-1-(4-methoxyphenyl)-2-(3,5-dimethoxyphenyl)-ethene (5a)

colourless oil; Rf (toluene) : 0,4; ¹H NMR (acetone-*d*₆): 3.66 (s, 6H), 3.77 (s, 3H), 6.34(t, J=2.16Hz, 1H), 6.429 (d, J=2.7Hz, 2H), 6.45 (d, J=12.4Hz, 1H), 6.556 (d, J=12.4Hz, 1H), 6.824 (m, 2H), 7.218 (m, 2H); ¹³C NMR (acetone-*d*₆): 55.418, 55.47, 100.085, 107.372, 114.387, 129.443, 130.315, 130.883 , 131.014, 140.307, 159.925, 161.706 ; MS (DCI/NH3) : *m*/*z* 271 (MH⁺); UV : λₘₐₓ= 286 nm ε = 25 500 M⁻¹.cm⁻¹

### (Z)-1-(4-chlorophenyl)-2-(3,5-dichlorophenyl)-ethene (5b)

colourless oil; Rf ( hexane): 0.63; ¹H NMR (acetone-*d*₆): 6.63 (d, J= 12.18Hz, 1H) 6.77 (d, J= 12.18Hz, 1H ), 7.19 (d, J=1.9 Hz, 2H), 7.23-7.36 (m, 5H); ¹³C NMR (acetone-*d*₆): 127.748, 128.087, 128.932, 129.467, 131.327, 132.398, 133.899, 135.494, 135.87, 141.375 MS (DCI/CH₄): *m*/*z*: 283 (MH⁺; 100), 311 (M....C₂H₅⁺; 46), 323 (M....C₃H₅⁺;3.6);UV : λₘₐₓ = 286 nm ε= 13 600 M⁻¹.cm⁻¹

### (Z)-1-(4-methoxyphenyl)-2-(3,5-difluorophenyl)-ethene (5c)

colourless oil; Rf ( toluene 1 / hexane 1) : 0.62; ¹H NMR (acetone-*d*₆): 3.788 (s, 3H), 6.497 ( d, J = 12.15 Hz, 1H), 6.706 ( d, J = 12.15 Hz, 1H), 6.819-6.88 (m, 5H), 7.19(m, 2H); ¹³C NMR (acetone-*d*₆): 55,506, 102.915, 112.3, 114.678, 127.067, 130.97, 133.103; MS (DCI/CH₄): *m*/*z*=227 (MH⁺-HF;30.37), 247 (MH⁺;100), 275 (M....C₂H₅⁺; 18.28), 287 (M....C₃H₅⁺); UV : λₘₐₓ= 291 nm ε= 14 000 M⁻¹.cm⁻¹

### (Z)-1-(4-fluorophenyl)-2-(3,5-difluorophenyl)-ethene (5d)

colourless oil; Rf (hexane) : 0.41; ¹H NMR (acetone-*d*₆): 6.62 ( d, J= 12.2Hz, 1H), 6.81- 6.9 (m,4H), 7.25-7.32( m, 2H); ¹³C NMR (acetone-*d*₆): 103.2, 112.4, 116.16, 128.86, 131.63,132.3; MS (DCI/CH₄): *m*/*z* = 215 (MH⁺-HF; 17), 234 (M⁺;100), 263 (M..C₂H₅⁺; 7), 275 (M....C₃H₅⁺; 0.45); UV : λₘₐₓ = 277 nm s= 6000 M⁻¹.cm⁻¹

### (Z)-1-(4-trifluoromethylphenyl)-2-(3,5-ditrifluoromethylphenyl)-ethene (5e)

colourless oil; Rf (hexane) : 0.39; ¹H NMR (acetone-*d*₆): 6.97 (d, J=12.2 Hz, 1H ), 7.05 (d, J=12.2 Hz, 1H ), 7.48 (m, 2H), 7.66 (m, 2H), 7.797 (s, 2H), 7.89 (s, 1H); ¹³C NMR (acetone-*d*₆): 121.697, 126.403, 130.089, 130.277, 133.211; MS (DCI/CH4): *m*/*z* = 365 (MH⁺-HF ;100), 385 (MH⁺ ;17.9), 413 (M....C₂H₅⁺; 15.5), 425 (M....C₃H₅⁺; 3.7); UV : λₘₐₓ = 276 nm ε= 9000 M⁻¹.cm⁻¹

### (Z)-1-(4-fluorophenyl)-2-(3,5-dimethoxyphenyl)-ethene (5f)

colourless oil; Rf (toluene): 0.59; ¹H NMR (acetone-*d*₆): 3.657 ( s, 6H), 6.362 ( t, J =2.25 Hz, 1H), 6.39 (d, J =2.2 Hz, 2H), 6.556 (d, J = 12.2 Hz, 1H ), 6.625 (d, J = 12.2 Hz, 1H ), 7.037 (m, 2H), 7.306 (m, 2H); ¹³C NMR (acetone-*d*₆): 55.425, 100.378, 107.421, 115.81, 130.068, 131.194, 131.647 ; MS (DCI/NH3): *m*/*z* = 259 (MH⁺; 100); UV : λₘₐₓ = 277 nm ε = 39 000 M⁻¹.cm⁻¹

### (Z)-1-(4-ethoxyphenyl)-2-(3,5-dimethoxyphenyl)-ethene (5g)

colourless oil; Rf (toluene): 0.5; ¹H NMR (acetone-*d*₆): 1.34 ( t, J = 6.96 Hz, 3H), 3.66(s,6H), 4.02 (q, J = 6.98 Hz, 2H), 6.337 (t, J =2.27 Hz, 1H), 6.428 (d, J =2.28 Hz, 2H), 6.44 (d, J= 12.28Hz, 1H ), 6.55 ( d, J= 12.28Hz, 1H ), 7.192(m, 2H), 7.225 (m, 2H); ¹³C NMR (acetone-*d*₆): 15.059, 55,414, 63.913, 100.073, 107.357 , 114.886, 129.355, 130.75, 130.926, 131.02, 140.321, 161.703; MS(DCI/NH3): *m*/*z* 285(M+1, 100); 302(MH⁺....NH3); UV : λₘₐₓ= 285 nm ε= 24 000 M⁻¹.cm⁻¹

### (Z)-1-(4-butoxyphenyl)-2-(3,5-dimethoxyphenyl)-ethene (5h)

colourless oil; Rf (toluene): 0.56; ¹H NMR (acetone-*d*₆): 0.952 (t, J= 7.27Hz, 3H), 1.47 (m,2H) 1.688-1.76 (m,2H), 3.662 (s, 6H), 3.968 (t, J= 7.1Hz, 2H), 6.339 (t, J=2.28 Hz, 1H), 6.435 (d, J=2.68 Hz, 2H), 6.44 (d, J= 12.3 Hz, 1H), 6.55 (d, J= 12.3 Hz, 1H), 6.815 (m, 2H), 7.209 (m, 2H); ¹³C NMR (acetone-*d*₆): 14.103, 19.866, 32.041, 55,413, 68.152, 100.053, 107.357, 114.918, 129.338, 130.154, 130.932, 131.013, 140.329, 159.416, 161.691 MS (DCI/NH3): *m*/*z* 313 (M+1, 100); UV : λₘₐₓ= 286 nm ε= 38 200 M⁻¹.cm⁻¹

### (Z)-1-(4-trifluoromethylphenyl)-2-(3,5-dichlorophenyl)-ethene (5i)

colourless oil; Rf (hexane): 0.48; ¹H NMR (acetone-*d*₆): 6.764 (d, J= 12.52 Hz, 1H), 6.891 (d, J= 12.52 Hz, 1H ), 7.195 (d, J=1.9 Hz, 2H), 7.3646 (t, J=1.93 Hz, 1H), 7.47 (m, 2H), 7.66 (m, 2H); ¹³C NMR (acetone-*d*₆): 126.216, 127.962, 128.147, 130.348, 132.251 MS (DCI/CH4): *m*/*z* 281 (MH⁺-HCl ; 8.5), 297 (MH⁺-HF; 100), 317 (MH⁺; 68.4), 345 (M....C₂H₅⁺; 28.96); UV : λₘₐₓ= 276 nm ε= 38 900 M⁻¹.cm⁻¹

### (Z)-1-(4-methoxyphenyl)-2-(3,5-dichlorophenyl)-ethene (5j)

colourless oil; Rf ( toluene/hexane): 0.75; ¹H NMR (acetone-*d*₆): 3.79 ( s, 3H), 6.476 (d, J= 12.12Hz, 1H ), 6.716 (d, J= 12.12Hz, 1H ), 6.857 ( m, 2H), 7.2 (m, 4H), 7.313 ( t, J=1.93 Hz, 1H); ¹³C NMR (acetone-*d*₆): 55.529, 114.7, 126.407, 127.332, 128.026, 133.384, 135.353 MS(DCI/NH3): *m*/*z* 278 (MH⁺ ;100), 296 (MH⁺ ....NH3; 16.24); UV : λₘₐₓ= 296 nm ε= 12 300 M⁻¹.cm⁻¹

### AhR and ER-mediated transactivation.

Cis and Trans isomers were then tested for transcriptional modulatory activity in a DRE-TK-CAT containing stable cell line treated with TCDD. All the compounds displayed antagonist activities on AhR.
To assay the β estrogenic properties of resveratrol, tests were performed with a stable cell line expressing an ERE-driven Luciferase reporter gene for ER-mediated transcriptional regulation. Results showed that the presence of oxygen atom on substituants yielded compounds with weak agonistic activities with regard to estradiol through ER. None of the other compounds displayed any antogonistic activities through ER. Results for both lines of experiments are summarized on Tables I and II.

### Table I : Characteristics of trans-Stilbenes derivatives.

mp°C : melting point. Ago : agonist. N.M. : not measurable. RBA : relative binding affinity of the compound for the given receptor (AhR or ER). The estimated *in vitro* affinity of resveratrol for the AhR (calculated as IC₅₀ ) was 6.10⁻⁶ M.R.T.A. : Residual Transcriptional Activity : This is calculated as the residual induction of the stably transfected DRE-TKCAT reporter gene when 10⁻⁹ M of TCDD is challenged by 10⁻⁷ M of the compound, expressed as average +/- SEM (n = 3 to 6 determinations).

| | | | | AhR | | ER | |
|---|---|---|---|---|---|---|---|
| Compounds | R₁ | R₂ | mp °C | RBA | T.A. | RBA | T.A. |
| Resveratol | 4'-OH | 3,5-OH | | 1 | Ant | 0.01 | Ago |
| 4a | 4'-OMe | 3,5-OMe | 55-57 | 21.0 | Ant | 0.03 | Ago |
| 4b | 4'-Cl | 3,5-Cl | 94-96 | 130.0 | Ant | N.M | N.M |
| 4c | 4'OMe | 3,5-F | 108-110 | 17.5 | Ant | N.M | N.M |
| 4d | 4'-F | 3,5-F | 47-48 | 39.1 | Ant | N.M | N.M |
| 4^{e} | 4'-CF₃ | 3,5-CF₃ | 99-100 | 52.1 | Ant | N.M | N.M |
| 4f | 4'-F | 3,5-OMe | 46-48 | 32.1 | Ant | N.M | N.M |
| 4g | 3',5'-OMe | 4-OEt | 54-55 | 39.8 | Ant | N.M | N.M. |
| 4h | 3',5'-OMe | 4-OBu | 54-56 | 8.1 | Ant | N.M | N.M. |
| 4i | 4'-CF₃ | 3,5-Cl | 98-100 | 791.0 | Ago | N.M | N.M. |
| 4j | 4'-OMe | 3,5-Cl | 62-64 | 112.0 | Ant | N.M | N.M. |
| 4k | 3'CF₃ | 3,5-Cl | 110-111 | 26.3 | Ant | N.M | N.M. |
| 4l | 3'-OMe | 3,5-Cl | 68-70 | 37.2 | Ant | N.M | N.M. |

### Table II: Characteristics of cis-Stilbenes derivatives.

mp°C: melting point. Ago: agonist. N.M.: not measurable. RBA: relative binding affinity of the compound for the given receptor (AhR or ER). The estimated in vitro affinity of resveratrol for the AhR (calculated as IC₅₀) was 6.10⁻⁶ M. R.T.A.: Residual Transcriptional Activity: This is calculated as the residual induction of the stably transfected DRE-TKCAT reporter gene when 10⁻⁹ M of TCDD is challenged by 10⁻⁷ M of the compound, expressed as average +/- SEM. (n = 3 to 6 determinations).

| | | | AhR | | | ER | |
|---|---|---|---|---|---|---|---|
| Compounds | R₁ | R₂ | mp °C | RBA | TA | RBA | TA |
| 5a | 4'-OMe | 3,5-OMe | oil | 2.1 | Ant | 0.06 | Ago |
| 5b | 4'-Cl | 3,5-Cl | oil | 12.4 | Ant | N.M. | N.M. |
| 5c | 4'-OMe | 3,5-F | oil | 7.2 | Ant | N.M. | N.M. |
| 5d | 4'-F | 3,5-F | oil | 2.6 | Ant | N.M. | N.M. |
| 5e | 4'-CF₃ | 3,5-CF₃ | oil | 2.7 | Ant | N.M. | N.M. |
| 5f | 4'-F | 3,5-OMe | oil | 1.7 | Ant | N.M. | N.M. |
| 5g | 3',5'-OMe | 4-OEt | oil | 2.5 | Ant | N.M. | N.M |
| 5h | 3',5'-OMe | 4-OBu | oil | 3.7 | Ant | N.M. | N.M. |
| 5i | 4'-CF₃ | 3,5-Cl | oil | 10.9 | Ant | N.M. | N.M. |
| 5j | 4'-OMe | 3,5-Cl | oil | 13.0 | Ant | N.M. | N.M. |
| 5k | 4'-OMe | 3,5-CF₃ | oil | 6.3 | Ant | N.M. | N.M. |
| 5l | 3'-OMe | 3,5-Cl | oil | 7.1 | Ant | N.M. | N.M. |

### Measurements of cytotoxicity

Cytotoxicity was evaluated of the A549 cell line. Resveratrol and its halogenated homologs were not toxic up to a 10 microM concentration.

### Cell culture and gene reporter assays.

The human adenocarcinoma breast cell line MCF-7 was obtained from the American Tissue Culture Collection (Rockville, USA). The cells were established by stably transfecting MCF-7 cells with the ERE-globin-tk-luc-SV-Neo plasmid and thus expressed luciferase in an estrogen dependent manner MCF-7-Luc. MCF-7 cells were grown routinely in RPMI 1640 medium and (MCF-7-Luc) cells in DMEM medium, supplemented with 5% FBS (Gibco BRL, Life Technologies, Cergy Pontoise, France). Cells were incubated at 37°C in a humidified 5% CO2 incubator. For experiments, cells were grown for 5 days in phenol red-free medium, containing 5% dextran-coated charcoal treated FCS (DCC-FCS). Medium was changed after 2 days. At day 5, cells were treated or not with compounds. Compounds were dissolved in ethanol.

### Luciferase assay:

For each condition, 15.103 cells were seeded per well in 12-well plates and treated, as described above, during 16 hours in a final volume of 0.5 mL. At the end of the treatment, cells were washed with PBS and lysed in 150 µl of lysis buffer (Promega, Charbonnières, France). Luciferase activity was measured using the luciferase assay reagent (Promega), according to the manufacturer's instructions. Protein concentrations were measured using Bradford technique, to normalize the luciferase activity data. For each condition, average luciferase activity was calculated from the data of 3 independent wells.

The stable cell line 47DRE bearing the TCDD-responsive CAT construct was previously described (3). It was routinely grown in DMEM medium supplemented with 10 % fetal calf serum (FCS), 4,5 g/L glucose, and 0.6 units/ml (10-6 M) insulin. Unless stated otherwise, cells were established 24 hours before any experiment in a modified medium (stripped condition) containing 5% Dextran-charcoal-treated newborn calf serum instead of 10 % FCS. All other components remained unchanged. Assays for Dioxin inducibility of the integrated DRE-TK-CAT construct were performed after a 48 hour treatment with Dioxin plus or minus resveratrol derivatives as described in the text. All chemicals applied to the cells were diluted in ethanol, control cells received ethanol alone. CAT expression was assayed on whole cell extracts (100 µg protein) with a CAT-ELISA assay (Roche, Meylan, France) according to supplier's specifications. Experiments were done in triplicate.

### Ligand binding competition assay (Aryl Hydrocarbon Receptor)

Experiments were conducted exactly as previously described by Savouret et al., (3). Briefly, binding competition was performed using female New Zealand rabbit liver cytosol as the receptor source. The cytosol was prepared at 4°C in Hepes 20 mM pH 7.6, EDTA 1.5 mM, glycerol 10 %, β-mercapto-ethanol 10 mM, with Complete protease cocktail (Roche, Meylan, France) by homogenisation in a Ultra-Turax homogenizer (Bioblock Scientific, Illkirch, France) followed with 20 strokes in a Dounce Homogenizer with the tight pestle. The homogenate was centrifuged 30 min at 20,000 g. The supernatant was centrifuged at 105,000 g for 65 min. The cytosol was aliquoted, snap-frozen in liquid nitrogen and kept at -80 °C. Cytosol aliquotes (4.5 ml) were thawed on ice, diluted ten-fold in Hepes 20 mM pH 7.6, 5 mM CaCl2, containing Complete protease cocktail and 10 mM mercapto-ethanol, and recentrifuged 30 min. at 105,000 g. The cleared cytosol dilution was adjusted to 0.85 mg proteins/ml. 930 µl of diluted cytosol were preincubated with the desired amounts of unlabeled competitors in ethanol solutions for 1 hour at 4°C. Subsequently, 2,3,7,8-tetrachloro (1,6-3H) dibenzo-p-dioxin (28 Ci/mmole) was added at 0.2 nM and incubation continued for 3 hours at 4°C. Non-displaceable binding was assessed by incubating the aliquotes with 70 µl of a 2% activated charcoal suspension in Hepes 20 mM pH 7.6 for 90 minutes at 4°C, followed by centrifugation at 15,000 g for 10 minutes. 500 µl of the supernatants were counted in 5 ml of Ultima Gold cocktail (Packard, Meridien, CT) in a Beckman liquid scintillation Counter (45 % counting efficiency). Binding competition assays were repeated at least twice for each competitor and each point was performed in triplicate.

### Ligand binding competition assay (Estrogen Receptor)

ER binding experiments were conducted using Estrogen Receptors extracted from MCF-7 cells as previously described (4). Briefly MCF-7 (from ATCC) were grown to 80% confluency in DMEM medium (GiBCO BRL) suplemented with 10 % FCS. Cells were then scraped, and washed twice with PBS. After centrifugation for 10 minutes at 1500 rpm, the cells were resuspended in TM buffer (20 mM Tris, HCl pH 7.4; sodium molybdate, 20mM). Cells were broken by freeze-thaw lysis of the cell pellets in an equal volume of TM buffer. Cytosolic receptors were prepared by a 105,000 g x 60 minutes centrifugation at 0°C, and then stored at -80°C. This cytosolic receptor solution was diluted to 60% in TM buffer, and then incubated with the corresponding ligand for 18 hr at 4°C in a volume of 100 µl with 50 µg of protein and 2 nM of [3H]Estradiol and two concentrations (1 and 10µM) of unlabeled test ligand. Assays were terminated by loading 65 µl of the incubate on a 1..2 ml SephadexTM LH-20 column equilibrated with the TM buffer. The flow through was collected and counted for radioactivity in ready safe scintillant (Beckman).

### Cytotoxicity Assays

A MTT colorimetric assay was employed as described in (5) according to the established procedure 1. Drug stock solutions were prepared in EtOH and the final solvent concentration was = 1% EtOH(v/v), a concentration without effect on cell replication. Drugs were tested at 10 M on the human tumor cell line panel constituted of human lung carcinoma (A-549). Cells were cultured at 37°C in RPMI-1640 medium with streptomycin/penicillin and 5% (v/v) FCS in a humidified atmosphere containing 5% CO2. Initial seeding densities varied among the cell lines to ensure a final absorbance reading in control cultures in the range of 0.7-1 A562 units. After the addition of the samples to the cell culture, the cells were incubated for 5 days before the MTT reagent was added. Each test was performed in duplicate and absorbance readings varied no more than 5%.

### Biological effects of the stilbene derivatives of the invention

### A. Dioxins and other AhR ligands

The biological effects of AhR ligands have been disclosed (ref. 1). Briefly, they are mediated by AhR is present in the cytosol of mammalian cells of almost all organs and tissues bound to heat shock protein 90 (hsp90). Upon binding to ligand, AhR dissociates from hsp90 and the ligand-AhR complex is translocated to the nucleus through association with a structurally related protein, the AhR nuclear translocator (Arnt). Inside the nucleus, the heteromeric ligand/AhR/Arnt complex regulates gene transcription by binding to DNA at dioxin-responsive enhancers (DRE) located within, or upstream of, a number of genes coding for phase 1 (cytochrome P-450) enzymes such as CYPIA1, 1A2, and 1B1 and phase 2 (detoxification) enzymes such as glutathione S-transferase Ya, aldehyde-3-dehydrogenase, and NAD(P)H:quinone oxidoreductase.

The most important phase 1 enzyme is CYP1A1 which is part of the aryl hydrocarbon hydrolase activity (AHH) and leads to the oxidative metabolism of AhR ligands, often to more pro-carcinogenic compounds. Phase 1 enzymes also increase the production of reactive oxygen (ROS) which have been shown to be associated with lipid peroxidation, oxidative DNA damage and other pathologic effects AhR ligands also induce phase 2 enzymes responsible for detoxification and excretion of environmental toxicants.

Therefore, a complex activation of gene transcription occurs following exposure to AhR ligands, which may initiate toxic effects as well as attempts to clear the ligand or its active metabolites from the body.

The prototype AhR ligand is 2,3,7,8-tetrachlorodibenzo-p-dioxin (TCDD or dioxin) which binds to the AhR with the highest affinity of all known compounds (Kd = 10^{-10 to -11} M in mice and 10⁻⁹ M in humans).

It results from the inventor's demonstration of the AhR inhibitory activity of stilbene derivatives that a variety of AhR ligand-induced genes can be repressed. This directs the uses of stilbene derivatives into a number of different areas. By searching gene databanks (such as EMBL and Genbank) for genes containing one or several putative dioxin responsive elements (DRE) presenting the consensus sequence TNGCGTG, or GNGCGTG in any orientation, preferentially in their promoter region.

Outside of the already known phase 1 and phase 2 enzymes, it was discovered that a number of genes contain DRE's and, thus are likely to be regulated by AhR ligands and resveratrol. A number of these genes are listed in two tables according to their relationship to distinct physiological or pathological events.

Table 3 lists various genes involved in hormonal control, lipid metabolism and inflammation, genetic or degenerative diseases, proliferative diseases and cancers.

Table 4 lists human viruses and related human proteins.

Table 3 : Human genes containing DRE's in their regulatory regions. The bovine prion gene is also shown. Cds : coding sequence.

| GENES | Access Nr | Sites | Location | Comments |
|---|---|---|---|---|
| Enzymes | | | | |
| inducible NOS | X97821 | 4 | promoter | |
| endothelial NOS | U24214 | 1 | promoter | |
| Cyclooxygenase 2 | U20548 | 1 | promoter | |
| PG H Synthase | U44805 | 1 | promoter | |
| PG endoperoxide synthase | M31812 | 1 | promoter | |
| 5 - Lipoxygenase | D64068 | 5 | promoter. cds | |
| 15 - lipoxygenase | M38191 | 6 | promoter. cds | |
| Phospholipase A2 | U63384 | 2 | promoter | |
| Thromboxane A2 receptor | U11239 | 1 | promoter | |
| | D15054 | | | |

| Disease-related proteins | | | | |
|---|---|---|---|---|
| [CAM (CD54) | M65001 | promoter | 2 | |
| Adenomatous polyposis protein | U02509 | 2 | promoter. cds | non-coding |
| Adenomatous polyposis protein | D13981 | 1 | supplementary | brain specific |
| | | | exon in 5'UTR | multiple variants |
| Adenomatous polyposis protein | D13980 | 1 | 5'UTR | Creutzfeld-Jacob |
| Human Prion protein | D00015 | 2 | cds | bovine gene |
| [Bovine Prion protein] | D26150 | 4 | intron 1 | obesity |
| Ob (leptin) | U43589 | 5 | promoter, cds | Alzheimer |
| presenilin | L76518 | 5 | promoter | Familial |
| STM2 | U50871 | 7 | promoter | Alzheimer |
| | | | | |
| GADD p153 | S40707 | 2 | promoter | DNA damage |

| Oncogenes | | | | |
|---|---|---|---|---|
| WT 1b | S77896 | 1 | promoter | Wilm' fumor |
| WNT-5A | U39837 | 3 | promoter | |
| PCNA | J05614 | 1 | promoter | apoptosis |
| p53 | J04238 | 17 | complete gene | cell cycle |
| p53 | J04238 | 1 | promoter | |
| c-Ha-Ras | M13221 | 1 | promoter | |
| c-jun | U60581 | 3 | promoter | |
| c-myc p64 | M13930 | 4 | promoter | |
| c-raf-1 | M38134 | 2 | promoter | |
| ATF3 | U37542 | 2 | | |

Table 4 : Viral genes or genomes containg DRE's UTR: untranslated region LTR: long terminal repeat. IE : immediate early. Cds : coding sequence.

| VIRAL GENE | ACCESS NO | LOCATION | SITES Nr | COMMENT |
|---|---|---|---|---|
| Cytomegalovirus IE-1 | M64941 | promoter | 1 | |
| HTLV-1 | S76263 | 5'LTR | 1 | |
| HTLV-1 | S76263 | TAX gene | 1 | |
| HTLV-4 | X06392 | 5'LTR | 2 | |
| HIV 1 isolate BRU (LAV-1) | K02013 | 3'LTR | 1 | repeatedly found in |
| | | | | all isolates |
| HIV 2 | U22047 | genome | 2 | |
| HIV 2 EHO | U27200 | genome | 1 | |
| CD 4 | U01066 | promoter | 1 | HIV receptor |
| CXCR4 | AF005058 | promoter. cds | 9 | HIV co-receptor |
| Human Papilloma Virus (HPV) | X52061 | genome lcr | 1 | long control region |
| HPV 11 | J04351 | genome | 2 | |
| HPV 16 | M33616 | genome | 1 | 3'flanking region |
| HPV 25 | M50264 | promoter | 1 | gene E6 |
| HPV 33, 47, 58, 59 | | genome | | |
| Human poliovirus 1 | X70509 | 5'UTR | 1 | |
| Human rhinovirus 1 B | D00239 | genome | 2 | |
| Human rhinovirus 2, 14, 89 | | genome | | |
| Herpes virus HSV1 | U18080 | genome | 1 | IE transactivator |
| Herpes virus HSV1 | M13885 | genome | 1 | gene |
| | | | | "a" segment |
| Hepatitis A virus | K02990 | genome | 1 | |
| Hepatitis B virus | X98077 | genome | 1 | |
| Hepatitis C virus | D30613 | genome | 4 | |
| Adenovirus type 2 | J01917 | genome | 21 | |
| Adenovirus type 12 | J01910 | genome | 1 | right terminal repeat |
| Adenovirus type 12 | X14757 | promoter | 1 | EIA gene |
| Adenovirus type 19A | X95259 | early region 3 | 1 | HLA binding protein |
| | | | | gene |
| | | genome | | EIA gene |
| Adenovirus type 41 | M18289 | cds | 1 | attachment protein |
| Human respiratory syncitial virus | M 17212 | | 1 | gene |
| | | genome: | | M1, M2 proteins |
| Human influenza virus | | multiple sites in | | matrix proteins, |
| all registered subtypes | | various genes | | hemagglutinin, |
| | | | | neuraminidase |
| | | | | RNA polymerase |

### B. Cardiovascular effects of AhR ligands

Cigarette smoke contains benzo[a]pyrene (BaP) and other AhR ligands such as polychlorinated dibenzo-p-dioxins (PCDD) in the tar fraction. It has been estimated that the amount of BaP in mainstream cigarette smoke is between 40 and 100 ng per cigarette and that up to 460 ng of BaP per hour can be inhaled by non-smokers in a smoking environment. Daily intake of PCDDs by smoking 20 cigarettes a day has been estimated to be 4.3 pg/kg body weight/day. In heavy smokers, daily exposure to BaP could be as high as 0.05 mg/kg in addition to other unquantifiable AhR ligand exposure from cigarettes and from the environment. The AhR ligands in cigarette smoke are present in high enough concentrations to induce CYP1A1 and AHH activity in the lungs placenta , kidney ovary and in the endothelial cells lining blood vessels AHH metabolism of BaP results in reactive carcinogenic intermediates which bind to DNA forming predominantly covalent adducts Cigarette smoking has been identified as the direct cause of at least eight different human cancers including lung, bladder, gastrointestinal tract, and leukemia, as well as ischemic heart disease . There is also epidemiologic evidence linking smoking with acceleration of acquired immune deficiency (AIDS) and with osteoporosis and increased risk of fractures in both men and women.

In the cardiovascular system, AhR ligands may be atherogenic by disrupting the functions of endothelial cells in blood vessels. AhR ligands have been shown to induce CYP1A1/AHH in porcine aortic endothelial cells with ED50 values of 180 nM for BaP and 0.015 nM for TCDD Stimulation of these phase I enzymes leads to increased ROS production which may cause endothelial cell membrane damage and lipid oxidation. Oxidized LDL formed by this mechanism undergoes unregulated uptake by a scavenger receptor in vascular endothelial cells leading to over-accumulation and the formation of "foam cells". It has also been demonstrated that AhR ligands can disrupt endothelial integrity and permeability, which could allow increased uptake of cholesterol-rich lipoprotein remnants into the arterial wall leading to atheroma formation. This effect was only seen with PCBs that bind to the AhR and induce CYP1A1. In addition, the same PCBs increased oxidative stress and lipid peroxidation in cultured endothelial cells, which may be the mechanism of cell injury, and which was correlated in time with increases in CYP1A1 induction.

### C. Activation of the progression of HIV and other viral infection by AhR ligands

AhR ligands are known to enhance the replication of viruses in animals, It is believed that activation of latent human immunodeficiency virus type 1 (HIV-1) expression by AhR ligands, is important for the progression of acquired immune deficiency disease (AIDS). It has been shown that dioxin and other AhR ligands activate HIV-1 expression by induction of CYP1A1 and by activation of the cellular transcription factor, nuclear factor kappa-B (NF-kappa B) The genes for both of these factors (CYP1A1, NF-kappa B), as well as the long terminal repeat (LTR) of the HIV gene promoter itself, contain DRE's consistent with their modulation by AhR ligands. Moreover, the two known entryways of HIV into lymphocytes, the CXCR4 chemokine receptor and the CD4 glycoprotein also present multiple DRE's in their promoters (see table 4)

### D . Skin disorders associated with dioxins exposure

AhR ligands, especially dioxins, are potent inducers of chloracne. In every industrial accident involving these environmental toxins, chloracne was considered the hallmark of dioxin intoxication and was consistently observed in the majority of exposed ndividuals. This acneiform eruptive skin condition occurs as a result of an altered pattern of lifferentiation of the basal cells of sebaceous glands and an altered rate of differentiation of keratinocytes, These cells express increasing concentrations of AhR luring ongoing differentiation and, moreover, AhR ligands themselves nhance keratinocyte terminal differentiation. It is likely that AhR ligands elicit specific skin responses trough activation of the AhR resulting in modified levels of gene expression in the skin.

### E Osteoporosis and exposure to AhR ligands

There are epidemiologic data to support cigarette smoking as a risk factor for osteoporosis in both men and women. The plausibility of this relationship is strengthened by the dose-response relationship between the number of cigarettes smoked per day and the decrease in bone mineral density and by the association between smoking and increased fracture risk. In addition, studies of twins discordant for cigarette smoking have demonstrated that bone density of women who smoked, or who smoked more heavily, was found to be significantly lower than that of their twin sisters. The pairs with the largest difference in smoking had the largest difference in bone density. The mechanism by which smoking and osteoporosis are related remains unknown. However, it is unlikely to be a simple direct effect since the relationship of ovarian steroids and bone turnover is so importart.
It is believed by the inventors that AhR ligands present in cigarette smoke, and in the environment, are responsible for loss of bone density through 3 different interconnected effects 1) An anti-estrogenic effect of AhR ligands in bone since AhR ligands have been shown to inhibit a broad spectrum of estrogen-induced responses in rodents and in breast cancer cell lines 2). A direct toxic effect of AhR ligands on bone. Both AhR and Arnt have been found to be expressed in bone tissue of mice during embryonic development. The effect of TCDD was examined on the organization of bone tissue in vitro using primary cultures of normal diploid calvarial-derived rat osteoblasts. It was found that TCDD inhibited formation of bone tissue-like multicellular nodules suggesting a direct association with reduced bone mass. 3). A direct toxic effect of AhR ligands on the ovary. There is compelling evidence for a direct toxic effect of AhR ligands on the ovary. Women who smoke undergo menopause at an earlier age (between 1 and 4 years earlier) than non-smokers. In addition, the incidence of ovarian cancer in women is highest in industrialized urban areas and in women working in rubber, electrical or textile industries where exposure to AhR ligands are increased compared to other industries. Experimental data in animals showing direct toxic effects of BaP and other AhR ligands on oocytes support these epidemiological studies.

### F. Resveratrol, the natural molecule.

The low rate of coronary heart disease in France compared to other western countries, despite the presence of similar risk factors (high animal fat intake, low exercise levels, and high rate of smoking) has been called the French paradox However, France was found to have the highest consumption of wine of any of the countries studied, leading to the speculation that wine may contain cardioprotective compounds. In addition to ethanol, which in moderate consumption appears to reduce mortality from coronary heart disease by increasing high-density lipoprotein cholesterol and inhibiting platelet aggregation. Two flavonoids (catechin, quercetin) and the tri-hydroxystilbene, resveratrol, inhibit the synthesis of thromboxane in platelets and leukotriene in neutrophils, and modulate the synthesis and secretion of lipoproteins in animals and in human cell lines. However, flavonoids should be avoided in the treatment of human diseases due to their numerous harmful side-effects.

Resveratrol (3,5,4'-trihydroxystilbene) is the parent compound of a family of molecules, including glucosides and polymers, existing in cis and trans configurations in a variety of plants classified as spermatophytes of which vines, peanuts and pines are the crime representatives, Resveratrol is produced by plants as a phytoalexin or antifungal agent. Resveratrol is usually present in red wine in concentrations between 1 and 8 mg/L (trans-resveratroi: 1 to 5 mg/L; and cis-resveratrol: 0.5 to 4 mg/L).

Another recent study demonstrated that resveratrol prevented chemical induction of pre-neoplastic lesions in a mouse mammary gland culture model and could slow down the growth of skin tumors which had been initiated in mice by a two step carcinogenic stimulus. This effect was proposed to act through the inhibition of cyclo-oxygenase and hydroperoxidase enzymes, by induction of phase 2 drug-metabolising enzymes, by anti-oxidant activity and by inducing differentiation of cancer cells.
Since resveratrol is highly Lipophilic, it is poorly absorbed from fruit and vegetables in the diet. However, it is readily soluble in alcohol, and is present in therapeutic levels in many red wines which prove to be a good delivery system. This fact may be an advantage for wine drinkers such as the majority of the adult French population, but means that abstainers and small children are not exposed to the protective effects of resveratrol against AhR ligands. In addition, since dioxin and other hydrophobic AhR ligands are also soluble in alcohol, an alcoholic delivery system will increase the absorption of the very toxins one is trying to antagonize. It is preferable for reseveratrol to be administrated as a dietary supplement, dissolved in a small volume of oil or other inocuous lipophilic solvent.

The discovery by the inventors of the potent anti-dioxin effects of resveratrol, has important medical and socioeconomic implications. It has been demonstrated that resveratrol, in concentrations easily obtained clinically, can inhibit dioxin-induced phase I enzymes activity as well as interleukin-I beta production and HIV promoter induction. It can therefore protect against a variety of diseases and toxic effects related to exposure to AhR ligands.

The obtained data show that the known AhR associations with immunosuppression, cancer and viral infections such as HIV-induced AIDS may be prevented by AhR antagonists such as resveratrol. In addition, the constant presence of DRE's in inflammatory related genes such as IgE, cytokines, chemokines, their receptors and the iNOS gene suggests a role of AhR and its ligands in inflammatory diseases. Examples of such iNOS-mediated diseases include atopic dermatitis, rheumatoid and osteo-arthritis, neurodegenerative diseases (multiple scleroris, amyotrophic, lateral sclerosis), diabetes, recurrent abortion, and likely, several others.The presence of DRE's in genes for chemokines and their receptors further supports the link between AhR ligands and inflammation and fever.

Finally, the presence of DREs in the intronic enhancer of the bovine Prion gene (responsible for bovine spongiformis encephalaitis and highly suspected to be involved in the new variant of human Creutzfeld-Jacob's disease) and in the promoter region of presenilin and STM2 genes (Alzheimer's disease) suggests that AhR ligands may aggravate these neurodegenerative conditions.

### BIBLIOGRAPHIC REFERENCES

1. Rowlands JC, Gustafsson J-A. (1997). Aryl hydrocarbon receptor-mediated signal transduction. Crit Rev Toxicol 27, 109-134.
2. Bock, K. W. (1994). Aryl hydrocarbon or dioxin receptor: biologic and toxic responses.Rev Physiol Biochem Pharmacol 125, 1-42.
3.Savouret, J. F.; Antenos, M.; Quesne, M.; Xu, J.; Milgrom, E.; Casper, R. F. *J Biol Chem* 2001, 276, 3054-3059.
4. Poirot, M.; De Medina, P.; Delarue, F.; Perie, J.J.; Klaebe, A.; Faye, J. C. *Bioorg Med Chem* 2000, 8, 2007-2016.
5. Alley, M.C.; Scudiero, D.A.; Monks, A.; Hursey, M. L.; Cserwinski, M. J.; Fine, D.L.; Abbott, B. J.; Mayo, J. G.; Shoemaker, R. H.; Boyd, M.R. Cancer Res 1988, 48, 589-601.

## Claims

1. Stilbene derivatives having formula I 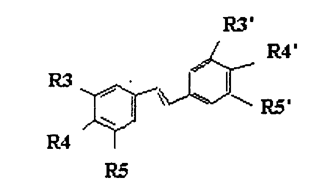 wherein
R3, R4 and R5 and R3', R4' and R5' are identical or different and represent H, OH, O-alkoxy or hal, said alkoxy group being a C1-C6 alkoxy and "hal" being F, Cl or CF₃, with the proviso that one of R4', R3 and R5 or R4, R3' and R5' does not represent OH, OCH₃ or OCH₂ CH₃ when the two other subtituents are both OH, OCH₃, OCH₂CH₃, respectively,
and the symmetrical derivatives.

2. The trans isomers of the stilbene derivatives of claim 1.

3. The trans isomers of claim 2, wherein R3 and R5 are hal.

4. The trans isomers of claim 3, wherein R3' or R4' is hal, alkoxy or hydroxy, and R5' is H.

5. The trans isomers of claim 3 or 4, wherein R3 and R5 are Cl.

6. The trans isomers of claim 5, wherein R3' is H, and R4' or R5' is Cl or methoxy.

7. The trans isomers of claim 2 selected in the group comprising (E)-1-(4'-trifluoromethylphenyl)-2-(3,5-ditrifluoromethylphenyl)-ethene, (E)-1-(4'-methoxyphenyl)-2-(3,5-dichlorophenyl)-ethene, and (E)-1-(4'-chlorophenyl)-2-(3,5-dichlorophenyl-ethene which bound to AhR with respective relative binding affinity of 52.1, 112.0 and 130.0, without detectable affinity for ER.

8. The symmetrical derivatives of the trans isomers of anyone of claims 3 to 7, particularly the derivatives with R3', R5' and R4 or R3', R5' are hal. and R4 is OH or alkoxy.

9. The use of the stilbene derivatives according to anyone of claims 1 t 8 as antagonists of AhR ligands.

10. Pharmaceutical compositions comprising an effective amount of at least one stilbene derivative according to anyone of claims 1 to 8, with a pharmaceutically acceptable carrier.

11. The pharmaceutically compositions of claim 10 in a form for administration by the oral, nasal, parenteral or tropical route.

12. The pharmaceutical compositions of claim 11, wherein said form is a gel, capsules, drops, syrup or alcohol syrup, for administration by the oral route, spray or drops for administration by the nasal route, solution for administration by the parenteral route, and cream, ointment, shampoo or lotion for application by the topical route, the vehicle comprising an oil or a pharmaceutically acceptable alcohol.

13. The pharmaceutical compositions of anyone of claims 10 to 12, said comprising then administration at a dosage from 0.1 mg to 5 g/day, especially from 20 to 200 mg/day and in particular from 10 to 100 mg/day.

14. The pharmaceutical compositions of anyone of claims 10 to 13 for the treatment of dermatitis, acne, psoriasis, hyperkeratotic lesions, eczema, or skin aging and wrinkling associated with common environmental exposure to AhR ligands.

15. The pharmaceutical compositions of anyone of claims 10 to 13 for preventing or avoiding the development of cold or flu symptoms related to viral infections aggravated by AhR ligands.

16. The pharmaceutical compositions of anyone of claims 10 to 13 for the prevention of AhR ligand-induced triggering of HIV (and other viruses) gene expression and progression of AIDS, particularly for the treatment of viral infections such as HIV-induced AIDS.

17. The pharmaceutical compositions of anyone of claims 10 to 13 for the prevention of prion-induced Spongiformis Encephalitis in humans and livestock.

18. The pharmaceutical compositions of anyone of claims 10 to 13 for the prevention of osteoporosis in reproductive age women and for the prevention and treatment of osteoporosis, alone or either in association with hormone replacement therapy or calcium and vitamin D in post-menopausal and elderly women.

19. The pharmaceutical compositions of anyone of claims 10 to 13 of inflammatory conditions caused by excessive nitric oxide and/or immunoglobulin E production such as: atopic dermatitis, rheumatoid and osteo-arthritis, neurodegenerative diseases (such as Alzheimer, multiple sclerosis, amyotrophic lateral sclerosis), diabetes.

20. The pharmaceutical compositions of anyone of claims 10 to 13 for reduction of fever associated with bacterial, viral, or allergic illnesses.

21. The pharmaceutical compositions of anyone of claims 10 to 13 for the treatment of obstetrical and gynecologic conditions such as endometriosis, fibroids (leiomyoma), pre-eclampsia and recurrent abortion.

22. Use of the stilbene derivatives according to anyone of claims 1 to 8, as food additive in powdered or liquid formula, cereal, and in canned food to prevent the toxic effects of environmental exposure to AhR ligands.

23. Use of the stilbene derivatives according to anyone of claims 1 to 8, for impregnating cigarette filters.
